# EUROPEAN PATENT SPECIFICATION

(11) **EP 1 748 760 B1**
(45) Date of publication and mention of the grant of the patent: **23.12.2009**
(21) Application number: 05748347.1
(22) Date of filing: 24.05.2005
(51) Int. Cl.: A61K 9/127, A61K 38/18, A61P 7/06

(54) **SUPERLOADED LIPOSOMES FOR DRUG DELIVERY**
SUPERBELADENE LIPOSOME FÜR DIE ARZNEIMITTELABGABE
LIPOSOMES SURCHARGES DESTINES A L'ADMINISTRATION DE MEDICAMENTS

(30) Priority: 24.05.2004 EP 04012224
(43) Date of publication of application: 07.02.2007
(73) Proprietor: Polymun Scientific Immunbiologische Forschung GmbH, 1190 Wien (AT)
(72) Inventor: KATINGER, Hermann, A-1190 Vienna (AT); WAGNER, Andreas, A-2500 Baden (AT); VORAUER-UHL, Karola, A-1220 Vienna (AT); KUNERT, Renate, A-2232 Deutsch-Wagram (AT); STROBACH, Stefanie, A-1230 Vienna (AT)
(74) Representative: Bogensberger, Burkhard
(86) International application number: PCT/EP2005/005577
(87) International publication number: WO 2005/115337

(56) References cited:
- US-A- 5 366 958
- US-A1- 2001 043 929
- US-A1- 2004 052 838
- XIAN-RONG QI ET AL: "EVALUATION OF LIPOSOMAL ERYTHROPOIETIN PREPARED WITH REVERSE-PHASE EVAPORATION VESICLE METHOD BY SUBCUTANEOUS ADMINISTRATION IN RATS" CHEMICAL AND PHARMACEUTICAL BULLETIN, PHARMACEUTICAL SOCIETY OF JAPAN, TOKYO, JP, vol. 43, no. 2, 1 February 1995 (1995-02-01), pages 295-299, XP000494628 ISSN: 0009-2363
- MORIYA H ET AL: "Pharmacokinetic and pharmacological profiles of free and liposomal recombinant human erythropoietin after intravenous and subcutaneous administrations in rats" PHARMACEUTICAL RESEARCH 1997 UNITED STATES, vol. 14, no. 11, 1997, pages 1621-1628, XP002337925 ISSN: 0724-8741

## Description

The present invention relates to liposomes containing erythropoietin or another pharmaceutically active compound being a glycoprotein or having a glycoprotein moiety with sialic acid groups at the end of its glycosilation sites. The invention further relates to a method of making the liposomes, to compositions containing them and to methods of using said liposomes.

### TECHNICAL BACKGROUND

Erythropoietin (EPO) is a well known glycoprotein involved in the synthesis of red blood cells. Pharmaceutical compositions comprising EPO together with human serum albumin (HSA) as a carrier are known in the art. However, as the use of HSA obtained from natural sources always bears a potential threat of transmitting infectious diseases, particularly viral infections, attempts have been made to replace HSA as carrier for parenteral EPO formulations.

EP 0 937 456 A1 discloses a liposome-based parenteral composition comprising erythropoietin together with liposomes in an aqueous dispersion, wherein the glycoprotein is not substantially incorporated within the liposomes but is essentially present in the aqueous phase outside the liposomes. The liposomes are being made by injecting an ethanolic lipid phase into an aqueous buffer using high speed homogenisation for making liposomes of less than 1 µm in diameter.

The Japanese patent JP823141 7 discloses another method of making a liposomal EPO-composition, wherein liposomes are prepared by reverse-phase evaporation to include EPO within the liposomes.

Moriya et al. (Pharm. Research, vol. 14, no. 11 (1997) p. 1621-1628) describe the encapsulation efficiency of the glycoprotein erythropoietin in liposomes. The encapsulation efficiency is in the range of 11.5 to 16.6%.

### SUMMARY OF THE INVENTION

While the methods known in the art may have certain advantages, it is the goal of the present invention to outperform the known compositions by providing liposomes that comprise a desired drug not only encapsulated in the interior, e.g. in the aqueous phase of the liposomes but also associated with or attached to the inside and/or outside of the liposomal membrane, resulting in liposomes loaded with a desired drug at an extent exceeding the usually or even theoretically obtainable load by passive drug inclusion, as predictable by numerical calulation based on the amounts of drug and lipid and the average size and/or total internal volume of the liposomes ("captured volume"). Thus the present invention relates to liposomes comprising molecules of at least one desired drug, wherein said drug molecules are present in an amount exceeding the drug load achieved by mere passive inclusion of said drug within the aqueous interior, i.e. without attachment of the drug to the membrane.

The desired drug is a pharmaceutically active compound which in a preferred embodiment is a glycoprotein or another bioactive compound having at least an oligosaccharide or polysaccharide moiety, wherein said glycoprotein or oligosaccharide or polysaccharide moiety comprises freely accessible, hence reactive sialic acid groups, e.g. at the end of its glycosilation sites.

Accordingly, it is an object of the invention to provide densely loaded liposomes wherein a desired drug is encapsulated within the liposomes and wherein a portion of the drug is also associated with or attached to the liposome membrane from the exterior, and typically also from the interior. In a preferred embodiment said drug is erythropoietin.

It is another object of the invention to provide a method of making the densely loaded liposomes.

It is yet another object of the invention to provide pharmaceutical compositions containing such densely loaded liposomes.

The principle of the invention is further described in the independent claims, while the various embodiments of the invention are the subject matter of the dependent claims.

### DETAILED DESCRIPTION OF THE INVENTION

More specifically, in a first embodiment the present invention relates to a liposome for drug delivery, wherein the liposome comprises molecules of at least one desired drug distributed within an aqueous phase in the interior of the liposome and wherein the liposome further comprises molecules of the same drug attached to either or both sides of the liposomal membrane. The attachment may be of a covalent or non-covalent nature, i.e. there may be a chemical bonding between drug and lipid or the attachment may be caused by adhesion forces other than a chemical bonding, including but not limited to van der Waals forces, hydrogen bridges, electrostatic forces, hydrophilic-hydrophobic interactions, affinity forces, and/or polar interactions and the like between drug and lipid molecules.

In another embodiment the invention relates to such a liposome, wherein at least a part of the drug molecules attached to the membrane are covalently linked to membrane lipids.

In a preferred embodiment the covalent linkage is a chemical bonding between a reactive functional group of a lipid and a functional group of the drug that is reactive with the functional group of the lipid. It is also preferred that the chemical bonding be of a nature such that it does not impede the intended physiological action of the drug upon administration to a human or animal body. In another preferred embodiment the chemical bonding is of nature such that it may be cleaved under the physiological conditions of a human or animal body upon administration.

Accordingly, in a specific embodiment the invention relates to such a liposome, wherein the functional group of the lipid is a hydroxyl group, which hydroxyl group may be a part of a polyvalent alcohol residue. Suitable polyvalent alcohol residues are sugar alcohol residues, particularly those naturally occurring in lipids such as glycerol residues and inositol residues. However, the lipid functional group providing for the attachment of a desired drug to the liposome membrane may also be a choline residue, such as, for example, in phosphatidyl choline (lecithin).

Where the lipid functional group is a hydroxyl group it is preferred that the reactive functional group of the drug is an acidic group, particularly an acidic group selected from the group consisting of a phosphoric acid residue, sulphuric acid residue, carbonic acid residue, and sialic acid residue. In that case the drug molecules or at least a part of the drug molecules that bear an acidic group will be covalently linked by ester bonding to at least a part of the lipid molecules that bear a hydroxyl group.

In another embodiment the invention relates to a liposome as described above, wherein the drug is a glycoprotein or has an oligosaccharide or polysaccharide moiety which glycoprotein or oligo- or polysaccharide moiety comprises at least one free reactive sialic acid group that is accessible for esterification by hydroxyl-containing lipids.

The lipid composition of the vesicle membrane of the liposomes typically comprises at least one lipid of natural or non-natural origin selected from the group consisting of phospholipids, glykolipids, ceramides, and derivatives of any one of these kinds of lipids.

Accordingly, in another specific embodiment the invention relates to an aforementioned liposome, wherein the liposome comprises phospholipids, particularly phospholipids selected from the group consisting of sphingophospholipids and glycerophospholipids, the sphingophospholipids comprising sphingomyelins and the glycerophospholipids comprising lecithins, kephalins, cardiolipins, phosphatidylinositols and phosphatidylinositol phosphates.

Where the liposomes comprise glykolipids instead of or in addition to phospholipids, said glykolipids are preferably selected from the group consisting of glykosphingolipids and glykoglycerolipids, the glykosphingolipids comprising cerebrosides, gangliosides, sulfatides, and the glykoglycerolipids comprising glykosylmonoglycerides and glykosyldiglycerides.

In a very specific embodiment, a liposome according to the present invention has a lipid composition, wherein the membrane comprises di-palmitoyl-phosphatidylcholine (DPPC), cholesterol, and egg phosphatidylglycerol (EPG) at a molar ratio of DPPC : cholesterol : EPG = 7 : 2 : 1, it being understood that this ratio may of course be varied qualitatively and/or quantitatively, as the case may be.

In another specific embodiment the drug for delivery encapsulated in and attached to the liposomes is erythropoietin.

In another embodiment, the invention relates to pharmaceutical compositions comprising a drug-loaded liposome as defined above together with a pharmaceutically acceptable carrier. The pharmaceutical composition may be in any suitable liquid, semi-liquid or solid form for administration, particularly for parenteral administration, such as in the form of an injection solution, a nasal spray, an inhalation liquid, a cream, a gel, an ointment, a suppository, or a lotion, in order to allow for topic or systemic parenteral administration.

The present invention further relates to a method of manufacture of a drug-loaded liposome comprising molecules of at least one desired drug distributed within an aqueous phase in the interior of the liposome and further comprising molecules of the same or of another drug attached to either or both sides of the liposomal membrane, the method comprising:
- providing a lipid phase in an organic solvent, wherein the lipid phase comprises at least one lipid fraction wherein each lipid molecule has at least one reactive functional group;
- providing an aqueous phase comprising a buffer solution and dissolved therein at least one desired drug, wherein at least one drug has a reactive functional group capable of reacting with a functional group of the lipids;
- feeding the lipid phase into the aqueous phase under conditions allowing for the formation of liposomes;
- optionally circulating the aqueous phase in a loop and repeating step c) in order to increase the efficiency of drug uptake by the liposomes; and
- harvesting drug-loaded liposomes,
wherein at least a part of the drug molecules are incorporated within the liposomes while another part of the drug molecules is attached to either or both sides of the liposomal membrane.

It shall be understood that terms such as "reactive functional group" or "reacting with a functional group", as used herein in connection with drugs and lipids, do not necessarily imply that the kind of reaction or reactivity be of a nature such that it results in a covalent chemical linkage but instead may also be of a nature such as to result in a more or less strong adhesion or attachment rather than a chemical linkage betweet lipid and drug, as outlined hereinbefore.

As mentioned above, the functional group of the lipids may be a hydroxyl group, particularly a hydroxyl group as a part of a polyvalent alcohol residue such as a sugar alcohol residue, preferably derived from naturally occurring sugar alcohols such as glycerol and inositol, or a choline group as in lecithins.

Also, as mentioned above, the reactive functional group of the drug may be an acidic group preferably selected from the group consisting of a phosphoric acid residue, sulfuric acid residue, carbonic acid residue, and sialic acid residue.

In one embodiment the method of manufacture comprises feeding the lipidic phase into the aqueous phase under conditions allowing for interaction, e.g. chemical reaction, of at least a part of said lipids carrying a reactive functional group with at least a part of said drug molecules bearing a reactive functional group.
Where the lipid functional group is a hydroxyl group and the drug functional group is an acidic residue, said conditions allowing for interaction and optionally chemical reaction comprise feeding the lipidic phase into the aqueous phase at a reaction temperature of about 25 to about 65°C and at a pH value of the aqueous phase of about 6 to about 8, whereupon at least a part of said drug molecules bearing a reactive functional group is being attached, optionally covalently linked by esterification, to at least a part of said lipids having functional groups.

In a more specific embodiment the drug is a glycoprotein or has an oligosaccharide or polysaccharide moiety which glycoprotein or oligo- or polysaccharide moiety comprises at least one reactive sialic acid group.

In yet another specific embodiment of the method of manufacture the lipidic phase comprises at least one lipid of natural or non-natural origin selected from the group consisting of phospholipids, glykolipids, ceramides, and derivatives of these lipids, while in a most specific embodiment the lipid composition of the lipidic phase is adjusted to comprise DPPC, cholesterol, and EPG at a molar ratio of 7 : 2 : 1, it being understood that this ratio may of course be widely varied qualitatively and/or quantitatively, as the case may be.

It is preferred that the lipidic phase be fed into the aqueous phase under pressure and under essentially shear-free conditions using the cross-flow injection technique disclosed in WO 02/36257 that allows for immediate and spontaneous formation of liposomes under extremely mild conditions, i.e. without using shear-force creating high speed homogenizers or similar means for creating high turbulence for vesicle formation.

In yet another embodiment the invention relates to a method of manufacture, wherein prior to the step of vesicle formation at least a part of the lipid fraction, or the entire lipid fraction, that contains lipids having at least one reactive functional group per lipid molecule, is being reacted with at least a part of those drug molecules that bear a reactive functional group, in order to attach or even covalently link drug molecules to membrane lipids. This pre-reaction is carried out prior to vesicle formation. Accordingly, in one embodiment the invention relates to a method of manufacture, wherein at least a part of the lipids of the lipidic phase is being attached, optionally covalently linked, to at least a part of the molecules of a desired drug prior to feeding the lipidic phase into the aqueous phase.

In a further embodiment, the invention relates to a method of manufacture of a drug-loaded liposome comprising molecules of at least one desired drug distributed within an aqueous phase in the interior of the liposome and further comprising molecules of the same or of another drug attached to either or both sides of the liposomal membrane, the method comprising:
- providing a lipidic phase in an organic solvent or as a dried film, wherein the lipidic phase comprises at least one lipid fraction wherein each lipid molecule has at least one reactive functional group;
- providing a first aqueous phase comprising a buffer solution;
- providing a second aqueous phase comprising a buffer solution and dissolved therein at least one desired drug, wherein at least one drug has a reactive functional group capable of attaching to or chemically reacting with a functional group of the lipids;
- combining the lipidic phase with the first aqueous phase under conditions allowing for the formation of liposomes;
- combining the liposomes formed with the second aqueous phase under conditions allowing for an uptake of at least a part of the drug molecules into the liposomes and allowing for interaction, optionally chemical reaction of at least a part of said lipids carrying a reactive functional group with at least a part of said drug molecules bearing a reactive functional group; and
- harvesting drug-loaded liposomes,
wherein at least a part of the drug molecules are incorporated within the liposomes while another part of the drug molecules is attached to either or both sides of the liposomal membrane.

Using this method it is preferred that the lipid functional group is a hydroxyl group and the drug functional group is an acidic residue and said conditions allowing for interaction, e.g. for a chemical reaction, comprise combining the liposomes with the second aqueous phase at a reaction temperature of about 25 to about 65°C and at a pH value of the second aqueous phase of about 6 to about 8, and incubating the resulting liposome suspension until at least a part of said drug molecules bearing a reactive functional group is being attached, optionally covalently linked by esterification, to at least a part of said lipids having functional groups.

In a further embodiment said method comprises an additional procedural step, wherein during or after incubation the liposome suspension is subjected to a further treatment for enhancing drug uptake into the liposomes, such treatment preferably being selected from the group consisting of sonication, electro-poration, vortexing, and gradient-driven transmembrane diffusion.

The covalent linkage may be an ester bonding. A suitable drug for "super-loading" liposomes with drug for delivery is erythropoietin.

The densely loaded liposomes of the present invention allow to considerably lower the dosage of parenteral application forms such as injection solutions, nasal sprays, or topical application forms such as creams, gels, ointments or lotions. Depending on the nature of the desired drug for delivery, the present liposome compositions allow adjustment of administration regimes of drugs in a way such as to reduce or substantially avoid undesired side-effects in human or animal recipients. Moreover, the liposomal formulations according to the present invention significantly increase and prolong bioavailability of pharmaceutically active compounds that are readily susceptible to degradation or damage under physiological conditions inside a human or animal body.

In addition to "super-loading" liposomes with a desired drug, it is another outstanding advantage of the present invention to offer the possibility to prepare liposomes containing two or more different drugs at reasonable amounts, wherein at least one drug is covalently or non-covalently attached to the liposome membrane while at least one other drug is present in the aqueous phase within the liposomes. Thus, using the concept of the present invention will enable a person of ordinary skill in the art to specifically design liposomes for the simultaneous delivery of two or more different drugs or drug components, where such delivery may be needed or beneficial, such as e.g. in combination therapies.

The present liposomes are preferably being made using the cross-flow injection technique disclosed in WO 02/36257, the contents of which shall be incorporated herein by reference. In brief, the method is based on injecting an organic lipid phase, typically an ethanolic lipid phase, into an aqueous phase through a tiny opening in the wall of a lipid phase piping and an adjacent opening in the wall of a second piping connected with said lipid phase piping and carrying an aqueous phase. The peculiarity with this method is that at the place of injection, i.e. the cross-flow injection module or mixing chamber, the piping conveying the lipidic phase is firmly connected to the piping of the aqueous phase in a way such that the pipings are in liquid connection with each other through a common orifice allowing organic liquid to enter, under pressure and in the form of a spray mist, the stream of the aqueous phase passing by said common orifice. No shear-force producing elements are present at the area of liquid intersection, i.e. where the organic lipid stream dashes in an approximately right angle into the aqueous stream, and yet liposomes are spontaneously formed in extraordinary quality and vesicle size distribution. This extremely mild method allows for the use of oxidation- or temperature-sensitive lipids as well as for the encapsulation of sensitive drugs that would otherwise be damaged or rendered inactive by cavitational phenomena or local overheating due to shear forces, such as may be the case e.g. in a homogenizer.

In addition to the method of passive incorporation of drugs into liposomes the present invention goes on to also actively attach desired drugs to the liposomal membrane, which typically is a unilamellar bilayer. In order to achieve this goal, i.e. to actively attach or associate a pharmaceutically active compound to the liposomal membrane, it is preferred that the lipid composition of the membrane is specifically selected in accordance with the desired drug(s) for delivery, i.e. to comprise lipids capable of interacting with the molecules of the desired drug(s), such as phospholipids of natural or non-natural origin having headgroups of the glycerol or inositol type, and to comprise other lipids, particularly of natural origin, which have only low amounts of lipids having such a headgroup.

Suitable lipids comprise sphingosins and derivatives thereof, cerebrosides and derivatives thereof, ceramides and derivatives thereof.

For active association or attachment of a desired drug to the liposomal membrane it is preferred that the liposomes be prepared in the presence of the desired drug, for instance erythropoietin, at a temperature of about 25 to about 65°C and at a pH around neutral, i.e. at a slightly basic or slightly acidic pH, preferably at a pH of from 6 to 8. The attachment, optionally including chemical linkage by esterification reaction between the sialic acid groups and the phospholipids, appears to work best at these temperature and pH ranges.

The liposomes according to the present invention typically comprise one lipid fraction selected from the group consisting of di-palmitoyl phosphatidylcholine (DPPC), egg phosphatidylcholine (EPC), soy phosphatidylcholine (SPC), ceramides and sphingosins as the main lipid fraction, together with at least one other lipid fraction selected from the group consisting of cholesterol and lipids having charged headgroups.

Using the aforementioned method of passive encapsulation by cross-flow injection of the lipid phase into the aqueous phase the following results were obtained:
a) by passive encapsulation
   encapsulation rates of up to 20 - 50 % by weight of the drug subjected to encapsulation were achieved, the results primarily depending on
   - the number of recirculation cycles performed with the drug-containing aqueous phase,
   - the preselected vesicle size of the developing liposomes, and
   - the lipid composition and lipid concentration of the membrane.

Such encapsulation rates are well in accordance with results from previous studies, e.g. for the liposomal inclusion of certain enzymes such as rhSOD;
whereas
b) by a combined passive and active loading of the liposomes according to the present invention
loading rates, i.e. encapsulation plus interior and exterior attachment to the liposomal membrane, of up to 100 % wt of the drug provided for loading were achieved, as confirmed by analysis of the filtrates after liposome loading. Typically; free drug was found in the filtrates in a concentration of 10 % wt or less, relative to the orginally supplied concentration.

Most of the experiments that have led to the present invention were carried out using erythropoietin as a desired drug to exemplify the drug-loading concept of the present invention. However, as mentioned hereinbefore the invention is applicable to any substances that may be both encapsulated within liposomes and attached to the liposomal membrane, preferably on either or both sides of the membrane. The attachment may be of a covalent or non-covalent nature, for instance an attachment by affinity, electrostatic interaction, van der Waals forces, hydrophilic and/or hydrophobic interaction, and the like, as known in the art. It is preferred, however, that the drug be covalently linked to a functional group of a lipid contained in the liposomal membrane. Where the functional group is of a polyol type, particularly of a sugar alcohol type, and typically is either inositol or glycerol, a desired drug will be most efficiently attached to the membrane if it contains a reactive acidic functional group such as a phosphoric acid, sulphuric acid, carbonic acid or sialic acid residue. Such an acidic reactive functional group may upon contact with a hydroxyl group of the lipid cause a covalent bonding between the lipid and the drug through esterification.

In order that the invention herein described be more fully understood, the subsequent examples are set forth.

### Example 1 (comparative example): Passive loading of liposomes with drug

a) Lipid composition
   DPPC : cholesterol : stearylamine = 7 : 2 : 1
b) Drug concentration
   2868 µg erythropoietin (EPO) per 2ml aqueous buffer (PBS)

The liposomes were manufactured according to the cross-flow injection method disclosed in WO 02/36257. Unilamellar bilayer liposomes having a mean vesicle diameter of about 170 nm were obtained and subsequently subjected to a filtration step using a ultra/diafiltration unit.

The filtrate was analyzed for free, i.e. non-encapsulated, drug the results being as follows:

| | |
|---|---|
| liposome retentate (2ml): | 71 1 µg EPO = 25% wt encapsulation; |
| total filtrates: | 2175 µg EPO = 75% wt non-encapsulated; |
| broken down into fractions: | filtrate F1 = 843 µg; filtrate F2 = 596 µg, |
| | filtrate F3 = 345 µg, filtrate F4 = 391 µg. |

In this example the use of the state-of-the art method for encapsulation of EPO within liposomes resulted in 25% wt inclusion of the drug, e.g. 356 µg drug per 1 ml liposome retentate. More generally, using this method of passive encapsulation of drugs typically resulted in inclusion rates ranging from about 25 to about 50% by weight, relative to the initially provided amount of drug.

This loading efficiency is well in line with previous studies using peptides or proteins such as recombinant human superoxide dismutase (rhSOD) as desired drugs for liposomal encapsulation.

### Example 2:

a) Lipid composition
   DPPC : cholesterol : EPG = 7 : 2 : 1
b) Drug concentration
   3375 µg Epo in 2ml aqueous buffer (PBS)

The liposomes were manufactured according to the cross-flow injection method disclosed in WO 02/36257. Unilamellar bilayer liposomes having a mean vesicle diameter of about 170 nm were obtained and subsequently subjected to a filtration step using a ultra/diafiltration unit.

The filtrate was analyzed for free, i.e. non-encapsulated drug the results being as follows:
liposome retentate (2ml): 3043 µg EPO = 90 % wt

| | |
|---|---|
| total filtrates: | 328 µg EPO = 10 % wt; |
| broken down into fractions: | filtrate F1 = 24 µg; filtrate F2 = 1 28 µg, |
| | filtrate F3 = 0-1 µg, filtrate F4 = 176 µg. |

In this example the cross-flow injection method for encapsulation of EPO within liposomes was carried out at a pH of 7.5 and at a temperature of 50°C, which resulted in 90% inclusion of the drug, corresponding to 1522 µg drug per 1 ml liposome retentate.

More generally, using this or an equivalent method of combined passive and active loading of liposomes with desired drugs by proper selection of lipids that allow for ready attachment, optionally involving covalent bonding of the drug to the liposome membrane (in addition to passive inclusion of the drug within the aqueous interior of the liposomes) will typically result in loading rates ranging from about 80 to about 100% by weight, relative to the initially provided amount of drug.

### Example 3: Combination of different drugs

Example 2 was repeated except that the aqueous phase further contained 3500 µg of rhSOD. After filtration, the liposome retentate comprised about 25% (approx. 820 µg per 2ml retentate) of the rhSOD and about 70% (approx. 2200 µg per 2 ml retentate) of EPO, while the balance of the amounts of drug was detected in the filtrates.

This example demonstrates that it is possible to "superload" liposomes even when using two different drugs at the same time. While the internal aqueous space of the liposomes was able to absorb, as expected, around 25 % wt of the total rhSOD offered, the liposomes were also able to absorb large amounts (approx. 70% wt) of the second drug, i.e. EPO. This was possible due to the interactions between the second drug and the EPG lipid fraction of the liposome membrane resulting in an attachment of said drug to said lipid fraction in the liposome membrane, plus a certain extent of passive inclusion within the aqueous interior of the liposome vesicles (not separately quantified herein).

The experiments described herein and further experiments (data not shown) impressingly demonstrated the superiority of the kind of active loading according to the present invention over conventional passive inclusion of a drug-containing aqueous phase. In fact, the present method of active loading and, particularly, of combined active and passive loading yielded liposomes loaded with drug in an amount of up to 200 % of the amounts that could be theoretically expected according to the captured volume of aqueous phase.

The highly concentrated liposome suspension harvested from the aqueous phase according to the present invention typically comprises about 0.5 - 2 mg drug per ml. It may be subsequently rediluted and/or formulated into usual solid, liquid or semi-liquid galenic preparations and pharmaceutical compositions for oral or parenteral application, particularly for intramuscular, intravenous, subcutaneous, cutaneous, intramucosal, intranasal, or intrapulmonary administration. Suitable compositions may be in the form of injection solutions, nasal sprays, inhalation liquids, creams, gels, ointments, suppositories, lotions, and the like, for topical or systemic administration.

## Claims

1. Liposome for drug delivery that comprises molecules of at least one desired drug distributed within an aqueous phase in the interior of the liposome, **characterized in that** the drug is a glycoprotein or has an oligosaccharide or polysaccharide moiety and comprises at least one reactive sialic acid group, the liposome further comprising molecules of the same drug attached to either or both sides of the liposomal membrane, wherein at least a part of the drug molecules is attached to the membrane through covalent linkage by chemical bonding or through non-covalent attachment.

2. Liposome according to claim 1, wherein said drug molecules are present in an amount exceeding a maximum drug load achievable by passive inclusion of said drug within the aqueous interior without attachment of drug molecules to the membrane.

3. The liposome according to claim 1 or 2, wherein said non-covalent attachment is by adhesion forces other than a chemical bonding, typically including at least one kind of adhesion forces selected from the group consisting of van der Waals forces, hydrogen bridges electrostatic forces, hydrophilic-hydrophobic interactions, affinity forces, and polar interactions, between drug and lipid molecules.

4. The liposome according to claim 1 or 2, wherein the membrane comprises lipids carrying a reactive functional group and attachment is between the reactive functional group of a lipid and a reactive sialic acid group of the drug.

5. Liposome according to claim 4, wherein the functional group of the lipid is a hydroxyl or a choline group.

6. Liposome according to claim 5, wherein the hydroxyl group is part of a polyvalent alcohol residue, preferably a part of a sugar alcohol residue selected from the group consisting of a glycerol residue and an inositol residue.

7. Liposome according to any one of claims 4 to 6, wherein at least a part of the drug is covalently linked to the lipids by an ester bonding.

8. Liposome according to any one of claims 1 to 7, wherein the membrane comprises at least one lipid of natural or non-natural origin selected from the group consisting of phospholipids, glykolipids, ceramides, and derivatives of these lipids.

9. Liposome according to claim 8, wherein the phospholipids are selected from the group consisting of sphingophospholipids and glycerophospholipids, the sphingophospholipids comprising sphingomyelins and the glycerophospholipids comprising lecithins, kephalins, cardiolipins, phosphatidylinositols and phosphatidylinositol phosphates.

10. Liposome according to claim 8 or 9, wherein the glykolipids are selected from the group consisting of glykosphingolipids and glykoglycerolipids, the glykosphingolipids comprising cerebrosides, gangliosides, sulfatides, and the glykoglycerolipids comprising glykosylmonoglycerides and glykosyldiglycerides.

11. Liposome according to any one of claims 1 to 10, wherein the membrane comprises DPPC, cholesterol, and EPG at a molar ratio of 7:2:1.

12. Liposome according to any one of claims 1 to 11, wherein the drug is erythropoietin.

13. Liposome according to any one of claims 1 to 12, further comprising at least one other drug.

14. Pharmaceutical composition comprising a drug-loaded liposome defined in any one of claims 1 to 13, together with a pharmaceutically acceptable carrier for oral or parenteral administration.

15. The pharmaceutical composition according to claim 14, in the form of an injection solution, a nasal spray, an inhalation liquid, a cream, a gel, an ointment, a suppository, or a lotion.

16. The pharmaceutical composition according to claim 14 or 15, for topic or systemic parenteral administration.

17. A method of manufacture of a drug-loaded liposome as claimed in claim 1, the method comprising:
- providing a lipidic phase in an organic solvent, wherein the lipidic phase comprises at least one lipid fraction wherein each lipid molecule has at least one reactive functional group;
- providing an aqueous phase comprising a buffer solution and, dissolved therein, at least one desired drug, wherein at least one drug is a glycoprotein or has an oligosaccharide or polysaccharide moiety and comprises at least one reactive sialic acid group;
- feeding the lipidic phase into the aqueous phase under conditions allowing for the formation of liposomes;
- optionally circulating the aqueous phase in a loop and repeating the feeding step in order to increase the efficiency of drug uptake by the liposomes; and
- harvesting drug-loaded liposomes,
wherein at least a part of the drug molecules are incorporated within the liposomes while another part of the drug molecules is attached to either or both sides of the liposomal membrane.

18. The method according to claim 17, wherein the functional group of the lipids is a hydroxyl or a choline group.

19. The method according to claim 18, wherein the hydroxyl group is part of a polyvalent alcohol residue, preferably a part of a sugar alcohol residue selected from the group consisting of a glycerol residue and an inositol residue.

20. The method according to any one of claims 17 to 19, wherein the lipidic phase is fed into the aqueous phase under conditions allowing for interaction of at least a part of said lipids carrying a reactive functional group with at least a part of said drug molecules bearing a reactive sialic acid group.

21. The method of claim 20, wherein the lipid functional group is a hydroxyl group and wherein said conditions allowing for interaction comprise feeding the lipidic phase into the aqueous phase at a reaction temperature of 25 to 65°C and at a pH value of the aqueous phase of 6 to 8, whereupon at least a part of said drug molecules bearing a reactive sialic acid group is covalently linked by esterification to at least a part of said lipids having functional groups.

22. A method of manufacture of a drug-loaded liposome as claimed in claim 1, the method comprising:
- providing a lipidic phase in an organic solvent or as a dried film, wherein the lipidic phase comprises at least one lipid fraction wherein each lipid molecule has at least one reactive functional group;
- providing a first aqueous phase comprising a buffer solution;
- providing a second aqueous phase comprising a buffer solution and, dissolved therein, at least one desired drug, wherein at least one drug is a glycoprotein or has an oligosaccharide or polysaccharide moiety and comprises at least one reactive sialic acid group;
- combining the lipidic phase with the first aqueous phase under conditions allowing for the formation of liposomes;
- combining the liposomes formed with the second aqueous phase under conditions allowing for an uptake of at least a part of the drug molecules into the liposomes and allowing for interaction, optionally chemical reaction, of at least a part of said lipids carrying a reactive functional group with at least a part of said drug molecules bearing a reactive sialic acid group; and
- harvesting drug-loaded liposomes,
wherein at least a part of the drug molecules are incorporated within the aqueous interior of the liposomes while another part of the drug molecules is attached to either or both sides of the liposomal membrane.

23. The method of claim 22, wherein the lipid functional group is a hydroxyl group and wherein said conditions allowing for interaction, optionally chemical reaction, comprise combining the liposomes with the second aqueous phase at a reaction temperature of 25 to 65°C and at a pH value of the second aqueous phase of 6 to 8, and incubating the resulting liposome suspension until at least a part of said drug molecules bearing a reactive sialic acid group is covalently linked by esterification to at least a part of said lipids having functional groups.

24. The method of claim 23, wherein during or after incubation the liposome suspension is subjected to a further treatment for enhancing drug uptake into the liposomes, such treatment preferably being selected from the group consisting of sonication, electroporation, vortexing, and gradient-driven transmembrane diffusion.

25. The method according to any one of claims 17 to 24, wherein the lipidic phase comprises at least one lipid of natural or non-natural origin selected from the group consisting of phospholipids, glykolipids, ceramides, and derivatives of these lipids.

26. The method according to any one of claims 17 to 25, wherein the lipidic phase comprises DPPC, cholesterol, and EPG at a ratio of 7 : 2 : 1.

27. The method according to any one of claims 17 to 26, wherein the lipidic phase is fed into the aqueous phase under pressure and essentially shear-free conditions using a cross-flow injection technique that allows for immediate and spontaneous formation of liposomes.

28. The method according to any one of claims 17 to 27, wherein at least a part of the lipids of the lipidic phase is covalently linked, preferably by an ester bonding, to at least a part of the molecules of the desired drug prior to feeding the lipidic phase into the aqueous phase.

29. The method according to any one of claims 17 to 28, wherein the desired drug is erythropoietin.

## Patentansprüche

1. Liposom zur Verabreichung eines Wirkstoffes, umfassend Moleküle mindestens eines gewünschten Wirkstoffes, verteilt in einer wässrigen Phase im Inneren des Liposoms, **dadurch gekennzeichnet, dass** der Wirkstoff ein Glykoprotein ist oder einen Oligosaccharid- oder Polysaccharidrest aufweist und mindestens eine reaktive Sialinsäuregruppe umfasst, wobei das Liposom des Weiteren Moleküle des gleichen Wirkstoffes umfasst, die entweder an einer oder an beiden Seiten der Liposommembran angeheftet sind, wobei wenigstens ein Teil der Wirkstoffmoleküle durch kovalente Verknüpfung mittels chemischer Bindung oder durch nicht kovalente Anlagerung an der Membran angeheftet ist.

2. Liposom gemäß Anspruch 1, wobei die Wirkstoffmoleküle in einer Menge vorliegen, die größer ist als eine maximale Wirkstoffbeladung, wie sie durch passiven Einschluss des Wirkstoffes im wässrigen Inneren ohne Anheftung von Wirkstoffmolekülen an der Membran erreicht werden kann.

3. Liposom gemäß Anspruch 1 oder 2, wobei die nicht kovalente Anlagerung durch Adhäsionskräfte außer einer chemischen Bindung erfolgt, typischerweise einschließend mindestens eine Art von Adhäsionskräften, ausgewählt aus der Gruppe bestehend aus van-der-Waals-Kräften, Wasserstoffbrückenbindungen, elektrostatischen Kräften, hydrophilhydrophoben Wechselwirkungen, Affinitätskräften und polaren Wechselwirkungen, zwischen Wirkstoff- und Lipidmolekülen.

4. Liposom gemäß Anspruch 1 oder 2, wobei die Membran Lipide umfasst, die eine reaktive funktionelle Gruppe tragen, und die Anheftung zwischen der reaktiven funktionellen Gruppe eines Lipids und einer reaktiven Sialinsäuregruppe des Wirkstoffes erfolgt.

5. Liposom gemäß Anspruch 4, wobei die funktionelle Gruppe des Lipids eine Hydroxyl- oder eine Cholingruppe ist.

6. Liposom gemäß Anspruch 5, wobei die Hydroxylgruppe Teil eines polyvalenten Alkoholrests ist, vorzugsweise ein Teil eines Zuckeralkoholrests, ausgewählt aus der Gruppe bestehend aus einem Glyzerinrest und einem Inositolrest.

7. Liposom gemäß einem der Ansprüche 4 bis 6, wobei mindestens ein Teil des Wirkstoffes durch eine Esterbindung kovalent mit den Lipiden verknüpft ist.

8. Liposom gemäß einem der Ansprüche 1 bis 7, wobei die Membran mindestens ein Lipid natürlichen oder nicht natürlichen Ursprungs umfasst, ausgewählt aus der Gruppe bestehend aus Phospholipiden, Glykolipiden, Ceramiden, und Derivaten dieser Lipide.

9. Liposom gemäß Anspruch 8, wobei die Phospholipide ausgewählt sind aus der Gruppe bestehend aus Sphingophospholipiden und Glycerophospholipiden, wobei die Sphingophospholipide Sphingomyeline und die Glycerophospholipide Lecithine, Kephaline, Cardiolipine, Phosphatidylinositole und Phosphatidylinositolphosphate umfassen.

10. Liposom gemäß Anspruch 8 oder 9, wobei die Glykolipide ausgewählt sind aus der Gruppe bestehend aus Glykosphingolipiden und Glykoglycerolipiden, wobei die Glykosphingolipide Cerebroside, Ganglioside und Sulfatide und die Glykoglycerolipide Glykosylmonoglyceride und Glykosyldiglyceride umfassen.

11. Liposom gemäß einem der Ansprüche 1 bis 10, wobei die Membran DPPC, Cholesterin und EPG in einem molaren Verhältnis von 7:2:1 umfasst.

12. Liposom gemäß einem der Ansprüche 1 bis 11, wobei der Wirkstoff Erythropoietin ist.

13. Liposom gemäß einem der Ansprüche 1 bis 12, des Weiteren umfassend mindestens einen weiteren Wirkstoff.

14. Pharmazeutische Zusammensetzung umfassend ein wirkstoffbeladenes Liposom, wie in einem der Ansprüche 1 bis 13 definiert, zusammen mit einem pharmazeutisch verträglichen Träger zur oralen oder parenteralen Verabreichung.

15. Pharmazeutische Zusammensetzung gemäß Anspruch 14 in Form einer Injektionslösung, eines Nasensprays, einer Inhalationsflüssigkeit, einer Creme, eines Gels, einer Salbe, eines Suppositoriums oder einer Lotion.

16. Pharmazeutische Zusammensetzung gemäß Anspruch 14 oder 15 zur topischen oder systemischen parenteralen Verabreichung.

17. Ein Verfahren zur Herstellung eines wirkstoffbeladenen Liposoms, wie beansprucht in Anspruch 1, umfassend:
- Bereitstellen einer Lipidphase in einem organischen Lösungsmittel, wobei die Lipidphase mindestens eine Lipidfraktion umfasst, in der jedes Lipidmolekül mindestens eine reaktive funktionelle Gruppe aufweist;
- Bereitstellen einer wässrigen Phase umfassend eine Pufferlösung und, darin aufgelöst, mindestens einen gewünschten Wirkstoff, wobei mindestens ein Wirkstoff ein Glykoprotein ist oder einen Oligosaccharid-oder Polysaccharidrest aufweist und mindestens eine reaktive Sialinsäuregruppe umfasst;
- Einbringen der Lipidphase in die wässrige Phase unter Bedingungen, die die Bildung von Liposomen gestatten;
- optional Zirkulieren der wässrigen Phase in einer Schleife und Wiederholen des Einbringungsschritts zur Steigerung der Effizienz der Wirkstoffaufnahme durch die Liposomen; und
- Ernten der wirkstoffbeladenen Liposomen,
wobei mindestens ein Teil der Wirkstoffmoleküle in den Liposomen eingeschlosssen wird, während ein weiterer Teil der Wirkstoffmoleküle an einer oder beiden Seiten der Liposommembran angeheftet wird.

18. Verfahren gemäß Anspruch 17, wobei die funktionelle Gruppe der Lipide eine Hydroxyl- oder eine Cholingruppe ist.

19. Verfahren gemäß Anspruch 18, wobei die Hydroxylgruppe Teil eines polyvalenten Alkoholrests ist, vorzugsweise ein Teil eines Zuckeralkoholrests, ausgewählt aus der Gruppe bestehend aus einem Glyzerinrest und einem Inositolrest.

20. Verfahren gemäß einem der Ansprüche 17 bis 19, wobei die Lipidphase unter Bedingungen in die wässrige Phase eingebracht wird, die eine Wechselwirkung von zumindest einem Teil der Lipide, die eine reaktive funktionelle Gruppe tragen, mit zumindest einem Teil der Wirkstoffmoleküle, die eine reaktive Sialinsäuregruppe tragen, ermöglichen.

21. Verfahren gemäß Anspruch 20, wobei die funktionelle Gruppe der Lipide eine Hydroxylgruppe ist und wobei die eine Wechselwirkung ermöglichenden Bedingungen das Einbringen der Lipidphase in die wässrige Phase bei einer Reaktionstemperatur von 25 bis 65°C und bei einem pH-Wert der wässrigen Phase zwischen 6 und 8 umfassen, woraufhin zumindest ein Teil der Wirkstoffmoleküle, die eine reaktive Sialinsäuregruppe tragen, durch Veresterung mit zumindest einem Teil der Lipide mit funktionellen Gruppen kovalent verknüpft wird.

22. Ein Verfahren zur Herstellung eines wirkstoffbeladenen Liposoms, wie beansprucht in Anspruch 1, umfassend:
- Bereitstellen einer Lipidphase in einem organischen Lösungsmittel oder als Trockenfilm, wobei die Lipidphase mindestens eine Lipidfraktion umfasst, in der jedes Lipidmolekül mindestens eine reaktive funktionelle Gruppe aufweist;
- Bereitstellen einer ersten wässrigen Phase umfassend eine Pufferlösung;
- Bereitstellen einer zweiten wässrigen Phase umfassend eine Pufferlösung und, darin aufgelöst, mindestens einen gewünschten Wirkstoff, wobei mindestens ein Wirkstoff ein Glykoprotein ist oder einen Oligosaccharid-oder Polysaccharidrest aufweist und mindestens eine reaktive Sialinsäuregruppe umfasst;
- Kombinieren der Lipidphase mit der ersten wässrigen Phase unter Bedingungen, die die Bildung von Liposomen ermöglichen;
- Kombinieren der gebildeten Liposomen mit der zweiten wässrigen Phase unter Bedingungen, die eine Aufnahme von zumindest einem Teil der Wirkstoffmoleküle in die Liposomen sowie eine Wechselwirkung, optional eine chemische Reaktion, von zumindest einem Teil der Lipide, die eine reaktive funktionelle Gruppe tragen, mit zumindest einem Teil der Wirkstoffmoleküle, die eine reaktive Sialinsäuregruppe tragen, ermöglichen; und
- Ernten der wirkstoffbeladenen Liposomen,
wobei zumindest ein Teil der Wirkstoffmoleküle im wässrigen Inneren der Liposomen eingeschlossen wird, während ein weiterer Teil der Wirkstoffmoleküle an einer oder beiden Seiten der Liposommembran angeheftet wird.

23. Verfahren gemäß Anspruch 22, wobei die funktionelle Gruppe der Lipide eine Hydroxylgruppe ist und wobei die eine Wechselwirkung, optional eine chemische Reaktion, ermöglichenden Bedingungen umfassen: das Kombinieren der Liposomen mit der zweiten wässrigen Phase bei einer Reaktionstemperatur von 25 bis 65°C und bei einem pH-Wert der zweiten wässrigen Phase zwischen 6 und 8, und Inkubieren der daraus resultierenden Liposomsuspension, bis zumindest ein Teil der Wirkstoffmoleküle, die eine reaktive Sialinsäuregruppe tragen, durch Veresterung mit zumindest einem Teil der Lipide mit funktionellen Gruppen kovalent verknüpft ist.

24. Verfahren gemäß Anspruch 23, wobei die Liposomsuspension während oder nach der Inkubation einer weiteren Behandlung zur Steigerung der Wirkstoffaufnahme in die Liposomen unterzogen wird, wobei eine solche Behandlung vorzugsweise ausgewählt ist aus der Gruppe bestehend aus Beschallung, Elektroporation, Vortexing und gradientengesteuerter transmembraner Diffusion.

25. Verfahren gemäß einem der Ansprüche 17 bis 24, wobei die Lipidphase mindestens ein Lipid natürlichen oder nicht natürlichen Ursprungs umfasst, ausgewählt aus der Gruppe bestehend aus Phospholipiden, Glykolipiden, Ceramiden, und Derivaten dieser Lipide.

26. Verfahren gemäß einem der Ansprüche 17 bis 25, wobei die Lipidphase DPPC, Cholesterin und EPG in einem Verhältnis von 7:2:1 umfasst.

27. Verfahren gemäß einem der Ansprüche 17 bis 26, wobei die Lipidphase unter Druck und unter im Wesentlichen scherfreien Bedingungen unter Verwendung einer Kreuzstrom-Injektionstechnik, die die sofortige und spontane Bildung von Liposomen ermöglicht, in die wässrige Phase eingebracht wird.

28. Verfahren gemäß einem der Ansprüche 17 bis 27, wobei zumindest ein Teil der Lipide der Lipidphase mit zumindest einem Teil der Moleküle des gewünschten Wirkstoffes kovalent verknüpft wird, vorzugsweise durch eine Esterbindung, bevor die Lipidphase in die wässrige Phase eingebracht wird.

29. Verfahren gemäß einem der Ansprüche 17 bis 28, wobei der gewünschte Wirkstoff Erythropoietin ist.

## Revendications

1. Liposome destiné à l'administration de médicaments, comprenant des molécules d'au moins un médicament souhaité, distribué, sous une phase aqueuse, à l'intérieur du liposome, **caractérisé en ce que** le' médicament est une glycoprotéine ou présente une teneur en oligosaccharide ou polysaccharide et comprend au moins un groupe acide sialique réactif, le liposome comprenant en outre des molécules du même médicament, attachées soit à l'un soit aux deux côtés de la membrane liposomale, dans lequel au moins une partie des molécules de médicament est attachée à la membrane par une liaison covalente, par liaison chimique ou par un attachement non covalent.

2. Liposome selon la revendication 1, dans lequel lesdites molécules de médicament sont présentes en une quantité supérieure à une charge médicamenteuse maximale pouvant être atteinte par inclusion passive dudit médicament dans l'intérieur aqueux, sans attachement des molécules de médicament à la membrane.

3. Liposome selon la revendication 1 ou 2, dans lequel ledit attachement non covalent est réalisé par des forces d'adhésion autres qu'une liaison chimique, comprenant typiquement au moins un type de forces d'adhésion sélectionné dans le groupe composé des forces de van der Waals, des forces électrostatiques des ponts hydrogène, des interactions hydrophiles-hydrophobes, des forces d'affinité et des interactions polaires, entre les molécules de médicament et de lipides.

4. Liposome selon la revendication 1 ou 2, dans lequel la membrane comprend des lipides portant un groupe fonctionnel réactif et l'attachement est réalisé entre le groupe fonctionnel réactif d'un lipide et un groupe acide sialique réactif du médicament.

5. Liposome selon la revendication 4, dans lequel le groupe fonctionnel du lipide est un groupe hydroxyle ou choline.

6. Liposome selon la revendication 5, dans lequel le groupe hydroxyle fait partie d'un résidu d'alcool polyvalent, de préférence une partie d'un résidu de sucre alcool sélectionné dans le groupé composé d'un résidu glycérol et d'un résidu inositol.

7. Liposome selon l'une quelconque des revendications 4 à 6, dans lequel au moins une partie du médicament est lié de manière covalente aux lipides par une liaison ester.

8. Liposome selon l'une quelconque des revendications 1 à 7, dans lequel la membrane comprend au moins un lipide d'origine naturelle ou non naturelle, sélectionné dans le groupe composé des phospholipides, glycolipides, céramides et des dérivés de ces lipides.

9. Liposome selon la revendication 8, dans lequel les phospholipides sont sélectionnés dans le groupe composé des sphingophospholipides et des glycérophospholipides, les sphingophospholipides comprenant des sphyngomyélines et les glycérophospholipides comprenant des lécithines, céphalines, cardiolipides, phosphatidylinositols et des phosphatidylinositol phosphates.

10. Liposome selon la revendication 8 ou 9, dans lequel les glycolipides sont sélectionnés dans le groupe composé des glycosphingolipides et des glycoglycérolipides, les glycosphingolipides comprenant des cérébrosides, des gangliosides, des sulfatides, et les glycoglycérolipides comprenant des glycosyl monoglycérides et des glycosyl diglycérides.

11. Liposome selon l'une quelconque des revendications 1 à 10, dans lequel la membrane comprend de la DPPC (dipalmitoylphosphatidylcholine), du cholestérol, et de l'EPG, selon un rapport molaire de 7:2:1.

12. Liposome selon l'une quelconque des revendications 1 à 11, dans lequel le médicament est l'érythropoïétine.

13. Liposome selon l'une quelconque des revendications 1 à 12, comprenant au moins un autre médicament.

14. Composition pharmaceutique comprenant un liposome chargé d'un médicament, défini selon l'une quelconque des revendications 1 à 13, conjointement avec un support acceptable du point de vue pharmaceutique pour une administration orale ou parentérale.

15. Composition pharmaceutique selon la revendication 14, se présentant sous la forme d'une solution d'injection, d'un produit nasal à pulvériser, d'un liquide pour inhalation, d'une crème, d'un gel, d'une pommade, d'un suppositoire, ou d'une lotion.

16. Composition pharmaceutique selon la revendication 14 ou 15, pour administration topique ou parentérale systémique.

17. Procédé de préparation d'un liposome chargé d'un médicament, tel que revendiqué selon la revendication 1, le procédé comprenant :
- la fourniture d'une phase lipidique dans un solvant organique, dans lequel la phase lipidique comprend au moins une fraction lipidique dans laquelle chaque molécule de lipide présente au moins un groupe fonctionnel réactif ;
- la fourniture d'une phase aqueuse comprenant une solution tampon et, dissoute dans celle-ci, au moins un médicament souhaité, dans lequel au moins un médicament est une glycoprotéine ou présente une teneur en oligosaccharide ou en polysaccharide et comprend au moins un groupe acide sialique réactif ;
- l'introduction de la phase lipidique dans la phase aqueuse, dans des conditions permettant la formation de liposomes ;
- la circulation optionnelle de la phase aqueuse en une boucle et la répétition de l'étape d'introduction, de manière à augmenter l'efficacité de l'assimilation de médicament par les liposomes ; et
- la récolte des liposomes chargés en médicament,
dans lequel au moins une partie des molécules de médicament sont incorporées dans les liposomes, tandis qu'une autre partie des molécules de médicament est attachée soit à l'un soit aux deux côtés de la membrane liposomale.

18. Procédé selon la revendication 17, dans lequel le groupe fonctionnel des lipides et un groupe hydroxyle ou choline.

19. Procédé selon la revendication 18, dans lequel le groupe hydroxyle fait partie d'un résidu d'alcool polyvalent, de préférence une partie d'un résidu de sucre alcool sélectionné dans le groupe composé d'un résidu glycérol et d'un résidu inositol.

20. Procédé selon l'une quelconque des revendications 17 à 19, dans lequel la phase lipidique est introduite dans la phase aqueuse dans des conditions permettant une interaction d'au moins une partie desdits lipides portant un groupe fonctionnel réactif avec au moins une partie desdites molécules de médicament portant un groupe acide sialique réactif.

21. Procédé selon la revendication 20, dans lequel le groupe fonctionnel lipidique est un groupe hydroxyle et dans lequel lesdites conditions permettant une interaction comprennent l'introduction de la phase lipidique dans la phase aqueuse, à une température de réaction de 25 à 65°C et à une valeur de pH de la phase aqueuse de 6 à 8, à la suite de quoi au moins une partie desdites molécules de médicament portant un groupe acide sialique réactif est liée de manière covalente, par estérification, à au moins une partie desdits lipides comprenant des groupes fonctionnels.

22. Procédé de préparation d'un liposome chargé d'un médicament, tel que revendiqué selon la revendication 1, le procédé comprenant :
- la fourniture d'une phase lipidique dans un solvant organique ou en tant que film séché, dans lequel la phase lipidique comprend au moins une fraction lipidique dans laquelle chaque molécule de lipide présente au moins un groupe fonctionnel réactif ;
- la fourniture d'une première phase aqueuse comprenant une solution tampon ;
- la fourniture d'une deuxième phase aqueuse comprenant une solution tampon et, dissoute dans celle-ci, au moins un médicament souhaité, dans lequel au moins un médicament est une glycoprotéine ou présente une teneur en oligosaccharide ou en polysaccharide et comprend au moins un groupe acide sialique réactif ;
- la combinaison de la phase lipidique avec la première phase aqueuse, dans des conditions permettant la formation de liposomes ;
- la combinaison des liposomes formés avec la deuxième phase aqueuse, dans des conditions permettant l'assimilation d'au moins une partie des molécules de médicament dans les liposomes et permettant l'interaction, en option la réaction chimique, d'au moins une partie desdits lipides portant un groupe fonctionnel réactif avec au moins une parties desdites molécules de médicament portant un groupe acide sialique réactif ; et
- la récolte des liposomes chargés en médicament,
dans lequel au moins une partie des molécules de médicament sont incorporées dans l'intérieur aqueux des liposomes, tandis qu'une autre partie des molécules de médicament est attachée soit à l'un soit aux deux côtés de la membrane liposomale.

23. Procédé selon la revendication 22, dans lequel le groupe fonctionnel lipidique est un groupe hydroxyle et dans lequel lesdites conditions permettant une interaction, en option une réaction chimique, comprennent la combinaison des liposomes avec la deuxième phase aqueuse, à une température de réaction de 25 à 65°C et à une valeur de pH de la deuxième phase aqueuse de 6 à 8, et l'incubation de la solution de liposome résultante, jusqu'à ce qu'au moins une partie desdites molécules de médicament portant un groupe acide sialique réactif soit liée de manière covalente, par estérification, à au moins une partie desdits lipides comprenant des groupes fonctionnels.

24. Procédé selon la revendication 23, dans lequel, durant ou après l'incubation, la suspension de liposome est soumise à un traitement supplémentaire, pour améliorer l'assimilation de médicament dans les liposomes, un tel traitement étant de préférence sélectionné dans le groupe composé de la sonication, l'électroporation, la vortexation et la diffusion transmembrane commandée par un gradient.

25. Procédé selon l'une quelconque des revendications 17 à 24, dans lequel la phase lipidique comprend au moins un lipide d'origine naturelle ou non naturelle, sélectionné dans le groupe composé des phospholipides, glycolipides, céramides et des dérivés de ces lipides.

26. Procédé selon l'une quelconque des revendications 17 à 25, dans lequel la phase lipidique comprend de la DPPC (dipalmitoylphosphatidylcholine), du cholestérol, et de l'EPG, selon un rapport molaire de 7:2:1.

27. Procédé selon l'une quelconque des revendications 17 à 26, dans lequel la phase lipidique est introduite dans la phase aqueuse sous pression et dans des conditions essentiellement exemptes de cisaillement, en utilisant une technique d'injection à courant croisé, permettant une formation immédiate et spontanée de liposomes.

28. Procédé selon l'une quelconque des revendications 17 à 27, dans lequel au moins une partie des lipides de la phase lipidique est liée de manière covalente, de préférence par une liaison ester, à au moins une partie des molécules du médicament souhaité, avant l'introduction de la phase lipidique dans la phase aqueuse.

29. Procédé selon l'une quelconque des revendications 17 à 28, dans lequel le médicament souhaité est l'érythropoïétine.
